# EUROPÄISCHE PATENTSCHRIFT

(11) **EP 1 061 867 B1**
(45) Veröffentlichungstag und Bekanntmachung des Hinweises auf die Patenterteilung: **20.04.2005**
(21) Anmeldenummer: 99902550.5
(22) Anmeldetag: 19.01.1999
(51) Int. Cl.: A61F 2/34

(54) **ORTHOPÄDISCHES KNOCHEN-IMPLANTAT MIT ANGEFORMTEM SCHNEIDGEWINDE**
ORTHOPEDIC BONE IMPLANT WITH A THREADING DIE SHAPED THEREON
IMPLANT OSSEUX ORTHOPEDIQUE AVEC FILETAGE COUPANT FORME SUR CE DERNIER

(30) Priorität: 22.01.1998 DE 19802215
(43) Veröffentlichungstag der Anmeldung: 27.12.2000
(73) Patentinhaber: Ortho Select, Implant Technology Gesellschaft für Medizinaltechnik mbH, 78582 Balgheim (DE)
(72) Erfinder: Imhof,Martin, 6343 Rotkreuz (CH)
(74) Vertreter: Patentanwälte Westphal, Mussgnug & Partner
(86) Internationale Anmeldenummer: PCT/EP1999/000275
(87) Internationale Veröffentlichungsnummer: WO 1999/037250

(56) Entgegenhaltungen:
- EP-A- 0 262 379
- EP-A- 0 318 679
- EP-A- 0 667 134
- CH-A- 677 072
- DE-A- 3 101 333
- US-A- 5 709 683

## Beschreibung

Die Erfindung betrifft ein orthopädisches Knochen-Implantat gemäß dem Oberbegriff des Anspruchs 1.

Knochen-Implantate dieser Gattung werden insbesondere als Endoprothesen oder zur Verankerung solcher Endoprothesen verwendet. Das Knochen-Implantat wird mittels seines Schneidgewindes in die Knochensubstanz eingeschraubt und in dieser verankert.

Bei einem aus der EP 0 318 679 B1 bekannten Knochen-Implantat ist das Schneidgewinde als Flachgewinde ausgebildet, dessen Flanken rechtwinklig zur Achse des Rotationskörpers verlaufen, so daß die Breite des Gewindeprofils vom Profilrücken gegen den Profilfuß konstant ist. Aufgrund der sich in Achsrichtung im Durchmesser erweiternden Form der Mantelfläche erweitert sich der Außendurchmesser des Schneidgewindes in Achsrichtung spiralig. Der Gewindegang des Schneidgewindes ist durch Lücken in aufeinanderfolgende Schneidzähne unterteilt. Der Profilrükken dieser Schneidzähne ist hinterschnitten, vorzugsweise hinterdreht oder hinterschliffen, so daß sein Radius gegenüber der in Drehrichtung vorderen Schneidfläche des Schneidzahnes abnimmt. Durch diese Ausbildung des Schneidprofils wird erreicht, daß beim Einschrauben des Knochen-Implantats die Gewindeflanken keinen axialen Druck und der Profilrücken keinen radialen Druck auf die Knochensubstanz ausüben. Solche axialen und/oder radialen Kräfte könnten zu Schädigungen der Knochensubstanz und zur Rißbildung führen.

Um bei diesem aus der EP 0 318 679 B1 bekannten Knochen-Implantat, die beim Eindrehen in den Knochen auftretende Reibung an den Gewindeflanken zu verringern, ist aus der EP 0 667 134 A1 bekannt, das Schneidgewinde mit einem sich ändernden Gewindeprofil auszubilden. Der Profilquerschnitt nimmt mit dem sich in Achsrichtung erweiternden Durchmesser des Schneidgewindes ab. Damit ergibt sich allerdings auch eine Reduzierung der Höhe des Gewindeprofils im Bereich mit größerem Gewindedurchmesser, so daß der Halt des Gewindes in der Knochensubstanz ebenfalls mit zunehmendem Gewindedurchmesser abnimmt.

Der Erfindung liegt die Aufgabe zugrunde, ein orthopädisches Knochen-Implantat der eingangs genannten Gattung so zu verbessern, daß das Knochen-Implantat mit geringer Reibung an den Gewindeflanken eingeschraubt werden kann und über die gesamte Gewindelänge eine konstante Verankerung in der Knochensubstanz gewährleistet.

Diese Aufgabe wird erfindungsgemäß gelöst durch ein Knochen-Implantat mit den Merkmalen des Anspruchs 1.

Vorteilhafte Ausführungen der Erfindung sind in den Unteransprüchen angegeben.

Der Grundgedanke der Erfindung besteht darin, das Gewindeprofil so auszubilden, daß sich seine Breite vom Profilrücken gegen den Profilfuß verringert. Da sich der Radius des Schneidgewindes bezogen auf die Rotationsachse kontinuierlich vergrößert, verschiebt sich das Gewindeprofil der in Drehrichtung vorderen Schneidfläche jedes Schneidzahnes geringfügig radial nach außen gegenüber dem vorangehenden Schneidzahn. Durch die gegen den Profilfuß abnehmende Breite des Gewindeprofils ergibt sich hieraus, daß jeder Schneidzahn des Schneidgewindes nur im Bereich des Profilrückens seiner in Drehrichtung vorderen Schneidfläche radial über das von dem vorangehenden Schneidzahn in die Knochensubstanz geschnittene Gewindeprofil hinausgreift, während die Gewindeflanken innerhalb des von dem vorangehenden Schneidzahn geschnittenen Gewindeprofils bleiben. Dadurch wird die Reibung zwischen den Gewindeflanken und der Knochensubstanz beim Eindrehen des Knochen-Implantats minimal.

Das Gewindeprofil kann dabei über die gesamte Gewindelänge eine konstante Höhe aufweisen, so daß auch die letzten Windungen des Schneidgewindes mit dem größten Durchmesser mit voller Profilhöhe in die Knochensubstanz eingreifen und einen guten Halt des Implantats gewährleisten. Die Profilhöhe des Schneidgewindes kann sich über die axiale Gewindelänge auch ändern. Nimmt die Profilhöhe mit zunehmendem Durchmesser ab, so verringert sich die Schneidwirkung in dem Endbereich mit großem Durchmesser. Das Eindrehen des Knochen-Implantats über die letzten Windungen wird dadurch erleichtert. Die letzten Windungen mit dem größten Durchmesser tragen dabei jedoch weniger stark zum Halt des Knochen-Implantats bei. In einer Ausführungsform weist das Schneidgewinde über die gesamte Gewindelänge ein konstantes Gewindeprofil auf. Jeder Schneidzahn des Schneidgewindes erzeugt dadurch an den Gewindeflanken einen minimalen Freischnitt für die Gewindeflanken des nachfolgenden Schneidzahnes. Dadurch wird die Reibung zwischen den Gewindeflanken und der Knochensubstanz beim Eindrehen des Knochen-Implantats weiter verringert.

In einer anderen Ausführung wird das Schneidgewinde zunächst mit über die gesamte axiale Länge des Knochen-Implantats konstantem Außendurchmesser erzeugt und erst dann überdreht, um den über den Verlauf der Gewindelänge spiralig ansteigenden Außenradius des Profilrückens zu erhalten. Diese Ausführung kann fertigungstechnik einfacher realisiert werden.

In vorteilhafter Weise ist das Gewindeprofil trapezförmig ausgebildet und weist einen flachen Profilrücken und gerade Gewindeflanken auf. Dies ist insbesondere für die Fertigung von Vorteil.

Im folgenden wird die Erfindung anhand eines in der Zeichnung dargestellten Ausführungsbeispiels näher erläutert. Es zeigen
- Figur 1: eine teilweise axial geschnittene Seitenansicht eines Knochen-Implantats,
- Figur 2: eine axiale Draufsicht auf das Implantat,
- Figur 3: drei verschiedene Gewindeprofil-Formen,
- Figur 4: eine Ansicht des Schneidgewindes in Richtung des Gewindeganges zur Verdeutlichung der Funktionsweise, und
- Figur 5: eine Fig. 4 entsprechende Darstellung für eine andere Ausführung der Gewindeprofilform.

In der Zeichnung ist als Ausführungsbeispiel für das orthopädische Knochen-Implantat eine Schale einer Gelenkpfanne gezeigt, welche für eine Hüftgelenks-Endoprothese in eine in dem Beckenknochen erzeugte Aushöhlung eingeschraubt wird. Das Implantat besteht vorzugsweise aus Titan. Die in die dargestellte Schale eingesetzte Gelenkpfanne besteht vorzugsweise aus Kunststoff. Die Ausbildung der Pfanne und die Befestigung der Pfanne in der Schale sind an sich bekannt und nicht Gegenstand der Erfindung. Eine Darstellung in der Zeichnung und eine Beschreibung sind daher nicht erforderlich.

Das als Schale für die Gelenkpfanne bestimmte Knochen-Implantat 10 hat die Form eines konischen Rocationskörpers mit einer konischen äußeren Mantelfläche 12. Auf der Mantelfläche 12 ist ein Schneidgewinde 14 einstückig angeformt, welches zum selbstschneidenden bzw. selbstfräsenden Eindrehen des Implantats 10 in die Höhlung des Beckenknochens dient.

Das Schneidgewinde 14 ist als eingängiges Gewinde ausgebildet, dessen Durchmesser sich in axialer Richtung entsprechend der konischen Ausbildung der Mantelfläche 12 spiralig erweitert. Die Steigung des Schneidgewindes 14 ist über die gesamte Länge des Schneidgewindes 14 konstant. Die Steigung beträgt dabei ein Mehrfaches, z. B. das Drei- bis Zehnfache der Breite des Gewindeprofils.

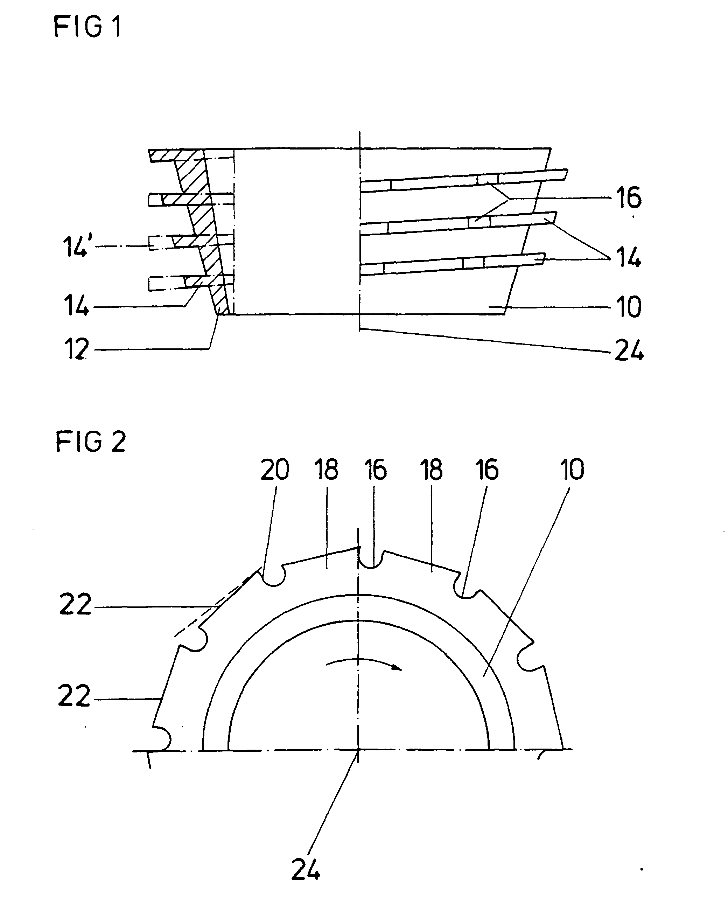

In dem Gewindegang des Schneidgewindes 14 sind in gleichem Abstand Lücken 16 vorgesehen, so daß der Gewindegang in einzelne aufeinanderfolgende Schneidzähne 18 unterteilt ist. Beim Eindrehen des Implantats 10 in der in Figur 2 durch einen Pfeil angezeigten Drehrichtung bilden die jeweils in Drehrichtung vorderen Flächen der Schneidzähne 18 eine Schneidfläche 20, mit welcher sich das Schneidgewinde 14 in die Knochensubstanz fräst.

Innerhalb der einzelnen Schneidzähne 18 ist der Profilrücken 22 hinterschliffen, wie dies in Figur 2 durch die gestrichelt eingezeichnete Tangente kenntlich gemacht ist, so daß die auf die Rotationsachse 24 bezogene radiale Höhe des Profilrückens 22 von der in Drehrichtung vorderen Schneidfläche 20 beginnend geringfügig abnimmt. Dadurch wird erreicht, daß beim Eindrehen des Implantats 10 der Profilrücken 22 hinter der fräsenden Schneidfläche 20 keinen radialen Druck auf die Knochensubstanz ausübt. Die Gewindeflanken 26 des Schneidgewindes 14 sind gerade ausgebildet, wobei die Teilflankenwinkel der Gewindeflanken 26 so gewählt sind, daß sich die in Achsrichtung gemessene Breite des Gewindeprofils von dem Profilrücken 22 gegen den Profilfuß an der Mantelfläche 12 verringert. Die Gewindeflanken 26 bilden zusammen mit dem flachen Profilrücken 22 somit ein trapezförmiges Gewindeprofil.

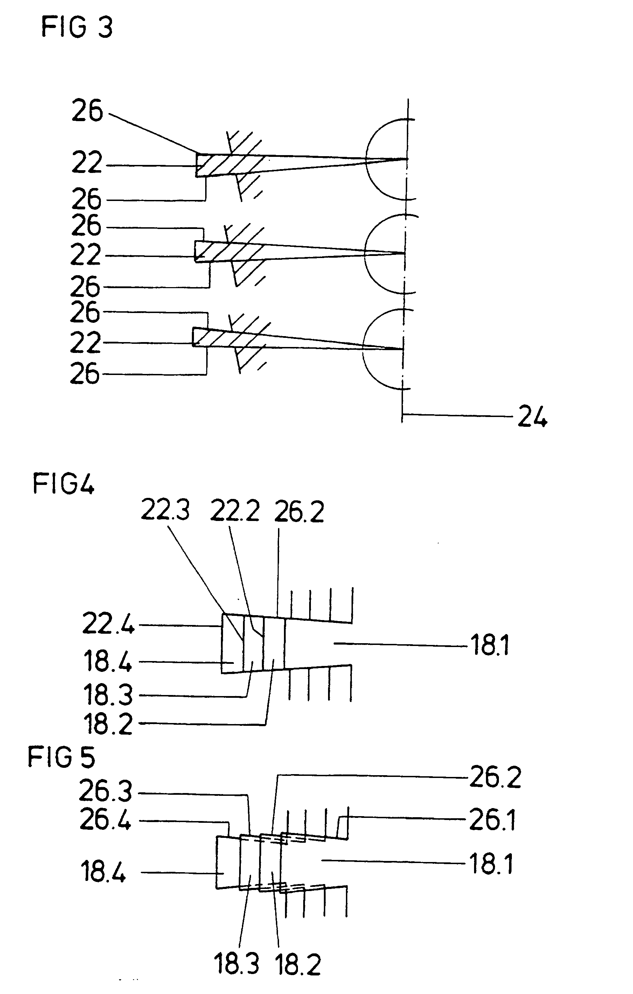

Um die Verringerung der Breite des Gewindeprofils vom Profilrücken 22 gegen den Profilfuß zu bewirken, kann eine Gewindeflanke 26 im Achsschnitt senkrecht zur Rotationsachse 24 verlaufen und die andere Gewindeflanke 26 unter einem von 90° Grad abweichenden Winkel hinterschnitten sein. In Figur 3 ist in dem oberen Beispiel die obere Gewindeflanke 26 senkrecht zur Rotationsachse 24 und die untere Gewindeflanke 26 hinterschnitten, während in dem unteren Beispiel die untere Gewindeflanke 26 senkrecht zur Rotationsachse 24 ist und die obere Gewindeflanke 26 hinterschnitten ist. Bevorzugt wird eine Ausführung, wie sie in Figur 3 im mittleren Beispiel dargestellt ist, bei welcher beide Gewindeflanken 26 symmetrisch gegenüber der Senkrechten zur Rotationsachse 24 hinterschnitten sind.

In einer ersten Ausführung wird das Schneidgewinde 14 in der weise hergestellt, wie dies in Fig. 1 durch die stichpunktierten Linien angedeutet ist. Die äußere Mantelfläche 12 des Implantats 10 wird konisch gedreht. Dabei wird das Schneidgewinde 14 zunächst in der Fig. 1 stichpunktierten Form 14' erzeugt. Das Schneidgewinde 14' weist einen über die gesamte Gewindelänge konstanten Außenradius auf. Die Gewindeflanken 26' sind von diesem konstanten Radius gegen einen fiktiven zylindrischen Gewindegrund hin geneigt, der in Fig. 1 strichpunktiert eingezeichnet ist. Anschließend wird das Schneidgewinde an seinem Außenumfang überdreht, so daß von dem Schneidgewinde 14' das in Fig. 1 ausgezogen gezeichnete Schneidgewinde 14 erhalten wird, bei welchem der Außenradius des Profilrückens 22 in axialer Richtung des Implantats 10 zunimmc.

Der Kegelwinkel der Mantelfläche 12 des Implantats 10 kann dabei mit dem Kegelwinkel eines die Profilrücken 22 des Schneidgewindes 14 einschließenden Hüllkegels übereinstimmen, so daß die Höhe der Profilrücken 22 bezogen auf die Mantelfläche 12 über die gesamte Gewindelänge konstant ist. Der Kegelwinkel der Mantelfläche 12 und der Kegelwinkel des Hüllkegels des Schneidgewindes 14 können auch voneinander abweichen, so daß die Profilhöhe des Schneidgewindes 14 bezogen auf die Mantelfläche 12 sich über die Gewindelänge ändert.

Die Funktionsweise des Schneidgewindes 14 beim Einschrauben des Knochen-Implantats 10 ist folgende:

Beim Eindrehen des Schneidgewindes 14 fräsen sich die Schneidzähne 18 aufeinanderfolgend in die Knochensubstanz, wobei aufgrund der konischen Ausbildung der Mantelfläche 12 jeder Schneidzahn 18 jeweils geringfügig radial gegenüber dem vorangehenden Schneidzahn 18 nach außen versetzt ist, wie dies aus Figur 1 ersichtlich ist. Zur Verdeutlichung ist dies in Figur 4 schematisch dargestellt. Es sind dort die Querschnitte gezeigt, die aufeinanderfolgende Schneidzähne 18.1, 18.2, 18.3 und 18.4 aus der Knochensubstanz ausfräsen, wobei die Form des Gewindeprofils dem mittleren Ausführungsbeispiel der Figur 3 entspricht. Die aufeinander folgenden Schneidzähne 18.1, 18.2, 18.3 und 18.4 fräsen aus der Knochensubstanz jeweils einen Gewindeprofil-Querschnitt aus, der von Schneidzahn zu Schneidzahn radial bezogen auf die Rotationsachse 24 nach außen verschoben ist. Die Gewindeflanken 26.1, 26.2, 26.3 und 26.4 kommen dabei jeweils zur Deckung mit den Gewindeflanken des von dem jeweils vorangehenden Schneidzahn ausgefrästen Gewindeprofil-Querschnitts. Nur der Bereich des Schneidzahns, der radial über den von dem vorangehenden Schneidzahn ausgefrästen Gewindeprofil-Querschnitt hinausragt, wird fräsend wirksam. Der restliche Bereich der Gewindeflanken 26 gleitet ohne wesentlichen Widerstand in dem von dem vorangehenden Schneidzahn 18 ausgefrästen Gewindeprofil.

In einer anderen Ausführung ist das Schneidgewinde 14 so ausgebildet, daß das Gewindeprofil über die gesamte Gewindelänge eine konstante Profilform aufweist. Im Gegensatz zu dem Ausführungsbeispiel der Fig. 1 und 4 weist das Schneidgewinde in dieser Ausführung über die gesamte Gewindelänge nicht nur eine konstante Höhe, sondern auch eine konstante Breite des Profilrückens 22 und eine konstante Breite des Profilfußes auf.

Die Funktionsweise des Schneidgewindes 14 in dieser Ausführung ist in Fig. 5 erläutert.

Der Schneidzahn 18.1 fräst aus der Knochensubstanz ein Querschnittsprofil aus, wie es in Figur 5 ausgezogen gezeigt ist. Der folgende Schneidzahn 18.2 befindet sich auf einem etwas größeren Radius bezogen auf die Rotationsachse 24, so daß die Knochensubstanz mit einem größeren Radius ausgefräst wird. Da das Querschnictsprofil konstant ist und die Gewindeflanken 26 hinterschnitten sind, ergibt sich eine geringere Breite der Schneidfläche 20 des jeweils nachfolgenden Schneidzahnes 18.2 in dem durch den vorangehenden Schneidzahn 18.1 in der Knochensubstanz ausgefrästen Querschnitt. Dies ist in Figur 5 erkenntlich durch die gestrichelt gezeichneten Gewindeflanken 26.2 des zweiten Schneidzahnes 18.2 gegenüber den Gewindeflanken 26.1 des ersten Schneidzahnes 18.1. Jeder Schneidzahn 18 erzeugt somit über den größten Teil seiner radialen Höhe einen geringen Freischnitt für die Gewindeflanken 26 des jeweils nachfolgenden Schneidzahnes 18. Dadurch wird der Reibungswiderstand zwischen den Gewindeflanken 26 des Schneidgewindes 14 und der Knochensubstanz beim Eindrehen des Implantates 10 gegenüber der Ausführung der Fig. 4 zusätzlich reduziert.

### Bezugszeichenliste

- 10: Implantat
- 12: Mantelfläche
- 14: Schneidgewinde
- 16: Lücken
- 18: Schneidzähne

- 20: Schneidfläche
- 22: Profilrücken
- 24: Rotationsachse
- 26: Gewindeflanken

## Patentansprüche

1. Orthopädisches Knochen-Implantat für eine HüftgelenkPfanne, mit einer äußeren Mantelfläche (12), die die Form eines sich in Achsrichtung im Durchmesser erweiternden Rotationskörpers hat, und mit einem auf der Mantelfläche (12) angeformten Schneidgewinde (14), wobei das Schneidgewinde (14) eine konstante Steigung aufweist, wobei sich der Außendurchmesser des Schneidgewindes (14) in Achsrichtung spiralig erweitert und wobei der Gewindegang durch Lücken (16) in Schneidzähne (18) unterteilt ist, die jeweils einen in Bezug auf die in Drehrichtung vordere Schneidfläche (20) des Schneidzahnes (18) hinterschnittenen Profilrücken (22) aufweisen,
**dadurch gekennzeichnet, dass** das Gewindeprofil des Schneidgewindes (14) von dem Profilrücken (22) gegen den Profilfuß in der Breite abnimmt.

2. Knochen-Implantat nach Anspruch 1, **dadurch gekennzeichnet, dass** das Gewindeprofil trapezförmig mit einem flachen Profilrücken (22) ausgebildet ist.

3. Knochen-Implantat nach Anspruch 1 oder 2, **dadurch gekennzeichnet, dass** das Gewindeprofil gerade Gewindeflanken (26) aufweist und wenigstens eine dieser Gewindeflanken (26) im Achsschnitt unter einem von 90° Grad abweichenden Winkel gegen die Rotationsachse (24) hinterschnitten ist.

4. Knochen-Implantat nach Anspruch 3, **dadurch gekennzeichnet, dass** beide Gewindeflanken (26) symmetrisch hinterschnitten sind.

5. Knochen-Implantat nach einem der Ansprüche 1 bis 4, **dadurch gekennzeichnet, daß** das Schneidgewinde (14) zumindest über einen Teil der Gewindelänge mit einem bezogen auf die Rotationsachse des Rotationskörpers gleichbleibenden Gewindeprofil mit konstantem Außenradius hergestellt ist und dieser Außenradius anschließend über die Gewindelänge konisch abgedreht ist.

6. Knochen-Implantat nach einem der Ansprüche 1 bis 4, **dadurch gekennzeichnet, daß** das Schneidgewinde (14) zumindest über einen Teil der Gewindelänge ein konstantes Gewindeprofil mit konstanter Breite des Profilrückens (22) und konstanter Profilhöhe bezogen auf die Mantelfläche aufweist.

7. Knochen-Implantat nach Anspruch 5, **dadurch gekennzeichnet, daß** das Schneidgewinde (14) über die gesamte Gewindelänge mit einem bezogen auf die Rotationsachse des Rotationskörpers gleichbleibenden Gewindeprofil mit konstantem Außenradius hergestellt ist und dieser Außenradius anschließend über die Gewindelänge konisch abgedreht ist.

8. Knochen-Implantat nach Anspruch 6, **dadurch gekennzeichnet, daß** das Schneidgewinde (14) über die gesamte Gewindelänge ein konstantes Gewindeprofil mit konstanter Breite des Profilrückens (22) und konstanter Profilhöhe bezogen auf die Mantelfläche aufweist.

## Claims

1. Orthopaedic bone implant for a hip joint socket, comprising an outer surface (12) which has the shape of a rotational body with a diameter which widens in the axial direction, and comprising a threading die (14) integrally formed on the outer surface (12), wherein the threading die (14) has a constant incline, wherein the outer diameter of the threading die (14) widens in a spiral-like manner in the axial direction, and wherein the thread path is divided by gaps (16) into cutting teeth (18) which each have a profile back (22) which is undercut with respect to the front cutting surface (20) of the cutting tooth (18) in the direction of rotation, **characterized in that** the thread profile of the threading die (14) decreases in terms of its width from the profile back (22) towards the profile base.

2. Bone implant according to Claim 1, **characterized in that** the thread profile is designed in a trapezoid-like manner with a flat profile back (22).

3. Bone implant according to Claim 1 or 2, **characterized in that** the thread profile has straight thread flanks (26) and at least one of these thread flanks (26) is undercut in axial section at an angle of other than 90° with respect to the axis of rotation (24).

4. Bone implant according to Claim 3, **characterized in that** both thread flanks (26) are undercut in a symmetrical manner.

5. Bone implant according to one of Claims 1 to 4, **characterized in that** the threading die (14), at least over part of the thread length, is produced with a thread profile which remains constant with respect to the axis of rotation of the rotational body and has a constant outer radius, and this outer radius is then conically turned over the thread length.

6. Bone implant according to one of Claims 1 to 4, **characterized in that** the threading die (14), at least over part of the thread length, has a constant thread profile with a constant width of the profile back (22) and a constant profile height with respect to the outer surface.

7. Bone implant according to Claim 5, **characterized in that** the threading die (14), over the entire thread length, is produced with a thread profile which remains constant with respect to the axis of rotation of the rotational body and has a constant outer radius, and this outer radius is then conically turned over the thread length.

8. Bone implant according to Claim 6, **characterized in that** the threading die (14), over the entire thread length, has a constant thread profile with a constant width of the profile back (22) and a constant profile height with respect to the outer surface.

## Revendications

1. Implant osseux orthopédique pour une cavité glénoïde de hanche, avec une enveloppe extérieure (12) qui a la forme d'un corps rotatif s'élargissant en diamètre dans la direction axiale, et avec un filetage coupant (14) formé sur l'enveloppe (12), le filetage coupant (14) présentant une inclinaison constante, le diamètre extérieur du filetage coupant (14) s'élargissant en spirale dans la direction axiale et le pas de filet étant subdivisé par des creux (16) en dents coupantes (18) qui présentent respectivement un dos de profil (22) contre-dépouillé par rapport à la face coupante (20), antérieure dans la direction de rotation, de la dent coupante (18),
**caractérisé en ce que**
le profil du filet du filetage coupant (14) diminue en largeur à partir du dos de profil (22) contre le pied de profil.

2. Implant osseux selon la revendication 1,
**caractérisé en ce que**
le profil du filet est de forme trapézoïdale avec un dos de profil (22) plat.

3. Implant osseux selon la revendication 1 ou 2,
**caractérisé en ce que**
le profil du filet présente des flancs de filet (26) droits et au moins l'un de ces flancs de filet (26) est contre-dépouillé dans la coupe axiale, sous un angle différent de 90°, contre l'axe de rotation (24).

4. Implant osseux selon la revendication 3,
**caractérisé en ce que**
les deux flancs de filet (26) sont contre-dépouillés symétriquement.

5. Implant osseux selon l'une des revendications 1 à 4,
**caractérisé en ce que**
le filetage coupant (14) est réalisé, tout au moins sur une partie de la longueur du filet, avec un profil de filet qui reste constant par rapport à l'axe de rotation du corps rotatif, avec un rayon extérieur constant, et ce rayon extérieur est ensuite réalisé coniquement au tour sur la longueur du filet.

6. Implant osseux selon l'une des revendications 1 à 4,
**caractérisé en ce que**
le filetage coupant (14) présente, tout au moins sur une partie de la longueur du filet, un profil constant avec une largeur constante du dos de profil (22) et une hauteur constante de profil sur l'enveloppe.

7. Implant osseux selon la revendication 5,
**caractérisé en ce que**
le filetage coupant (14) est réalisé, sur toute la longueur du filet, avec un profil de filet qui reste constant par rapport à l'axe de rotation du corps rotatif, avec un rayon extérieur constant, et ce rayon extérieur est ensuite réalisé coniquement au tour sur la longueur du filet.

8. Implant osseux selon la revendication 6,
**caractérisé en ce que**
le filetage coupant (14) présente, sur toute la longueur du filet, un profil constant avec une largeur constante du dos de profil (22) et une hauteur constante de profil par rapport à l'enveloppe.
